# EUROPEAN PATENT SPECIFICATION

(11) **EP 3 033 989 B1**
(45) Date of publication and mention of the grant of the patent: **24.04.2019**
(21) Application number: 15194447.7
(22) Date of filing: 13.11.2015
(51) Int. Cl.: A61B 5/00

(54) **OBJECT INFORMATION ACQUIRING APPARATUS AND CONTROL METHOD THEREFOR**
OBJEKTINFORMATIONSERFASSUNGSVORRICHTUNG UND STEUERUNGSVERFAHREN DAFÜR
APPAREIL D'ACQUISITION D'INFORMATIONS D'OBJET ET SON PROCÉDÉ DE COMMANDE

(30) Priority: 15.12.2014 JP 2014252879
(43) Date of publication of application: 22.06.2016
(73) Proprietor: Canon Kabushiki Kaisha, Tokyo 146-8501 (JP)
(72) Inventor: YAMAMOTO, Hiroshi, Tokyo 146-8501 (JP)
(74) Representative: TBK

(56) References cited:
- EP-A1- 2 868 266
- JP-A- 2012 179 348
- US-A1- 2013 312 526

## Description

### BACKGROUND OF THE INVENTION

### Field of the Invention

The present invention relates to an object information acquiring apparatus and a control method therefor.

### Description of the Related Art

Photoacoustic tomography (PAT) is a method for determining optical characteristic values for the inside of an object such as an organism. When radiated to an organism, pulsed light generated by a light source propagates through the organism while diffusing. A light absorber in the organism absorbs the propagated light to generate a photoacoustic wave (typically an ultrasonic wave). The photoacoustic wave is received via a probe (transducer) and the received signal is analyzed to allow acquisition of an initial sound pressure distribution attributed to the light absorber in the organism. Based on the initial sound pressure distribution and the distribution of light in the organism, optical characteristic values such as an absorption coefficient distribution can be acquired.

When the object is the breast, holding the breast using a curved holder applies a lower pressure to the breast than holding the breast using a flat holder, reducing a burden on the subject. Japanese Patent Application Laid-open No. 2012-179348 discloses an example where the breast is held using a cup-shaped holding portion and where a light irradiator and a probe integrally scan the cup to acquire a photoacoustic signal from the breast.

Patent Literature 1: Japanese Patent Application Laid-open No. 2012-179348

### SUMMARY OF THE INVENTION

However, in the apparatus in Japanese Patent Application Laid-open No. 2012-179348, the distance between the light irradiator and the holder (that is, the distance light travels before reaching the object) varies depending on the scan position of the light irradiator. Normally, an acoustic matching material (in Japanese Patent Application Laid-open No. 2012-179348, water) that allows acoustic coupling to be achieved needs to be arranged between the light irradiator and the holder. Light with a wavelength of 750 nm is decayed at a rate of approximately 2.6%/cm while propagating underwater. Therefore, in Japanese Patent Application Laid-open No. 2012-179348, when the distance between the light irradiator and the holder varies by 5 cm during the process of scanning by the light irradiator, the optical amount of light radiated to the object varies approximately by 12%. In this regard, the initial sound pressure distribution inside the body is proportional to the optical amount of light radiated to the object. Thus, when the optical amount of irradiation light varies depending on the scan position, the measurement accuracies of the initial sound pressure distribution and the absorption coefficient distribution decreases.

With these problems in view, the present invention is developed to reduce a variation in the optical amount of light radiated to an object in photoacoustic measurement in which a light irradiator moves.

The present invention in its first aspect provides an object information acquiring apparatus as specified in claims 1 to 13.

The present invention in its second aspect provides a control method for an object information acquiring apparatus as specified in claim 14.

The present invention enables a reduction in a variation in the optical amount of light radiated to the object in photoacoustic measurement in which the light irradiator moves.

Further features of the present invention will become apparent from the following description of exemplary embodiments with reference to the attached drawings.

### BRIEF DESCRIPTION OF THE DRAWINGS

FIG. 1A is a schematic diagram illustrating a configuration of Embodiment 1;
FIG. 1B is a diagram illustrating a process flow in Embodiment 1;
FIG. 2 is a diagram illustrating a configuration in Embodiment 2;
FIG. 3 is a diagram illustrating a configuration in Embodiment 3;
FIG. 4A is a diagram illustrating a configuration in Embodiment 4;
FIG. 4B is a diagram illustrating a process flow in Embodiment 4;
FIG. 5A is a diagram illustrating a configuration in Embodiment 5;
FIG. 5B is a diagram illustrating a process flow in Embodiment 5; and
FIG. 6 is a diagram illustrating Embodiment 6.

### DESCRIPTION OF THE EMBODIMENTS

Preferred embodiments of the present invention will be described below with reference to the drawings. However, the dimensions, materials, shapes, and relative arrangements of components described below should be changed as needed according to the configuration of an apparatus or conditions to which the invention is applied, and are not intended to limit the scope of the present invention to the following description.

The present invention relates to a technique for detecting an acoustic wave propagating from an object and generating and acquiring characteristic information on the inside of the object. Therefore, the present invention is considered to be an object information acquiring apparatus or a control method therefor or an object information acquiring method and a signal processing method. The present invention is also considered to be a program that allows an information processing apparatus with a hardware resource such as a CPU, or a storage medium that stores the program.

The object information acquiring apparatus in the present invention includes an apparatus utilizing a photoacoustic tomography technique to irradiate an object with light (electromagnetic wave) and receive (detect) an acoustic wave generated at and propagating from a particular position inside the object or on a surface of the object in accordance with a photoacoustic effect. Such an object information acquiring apparatus obtains characteristic information on the inside of the object in the form of image data, etc., based on photoacoustic measurement and may thus be referred to as a photoacoustic imaging apparatus.

Characteristic information in the photoacoustic apparatus includes the distribution of sources of acoustic waves resulting from light irradiation, an initial sound pressure distribution inside the object, an optical energy absorption density distribution and an absorption coefficient distribution derived from the initial sound pressure distribution, and a concentration distribution of a substance included in the tissue. Specifically, the characteristic information includes an oxidation and reduction hemoglobin concentration distribution, a blood component distribution such as an oxygen saturation distribution which is determined from the oxidation and reduction hemoglobin concentration distribution, or a distribution of fat, collagen, or moisture. The characteristic information may be obtained in the form of distribution information at each position in the object instead of numerical data. That is, object information may be the distribution information such as the absorption coefficient distribution or the oxygen saturation distribution.

The acoustic wave as used herein typically refers to an ultrasonic wave and includes an elastic wave referred to as a sound wave or an acoustic wave. An acoustic wave resulting from a photoacoustic effect is referred to as a photoacoustic wave or an optical ultrasonic wave. An electric signal into which the acoustic wave is converted by probes is also referred to as an acoustic signal.

### [Configuration of the Photoacoustic Apparatus]

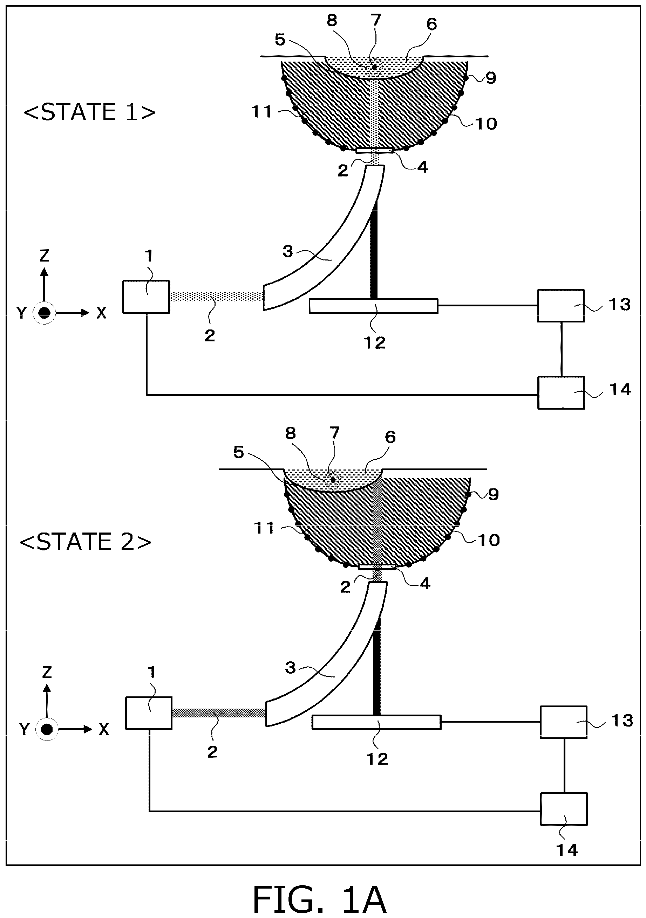

The photoacoustic apparatus acquires characteristic information on the inside of the object using the photoacoustic tomography technique. The following description is given with reference to reference numerals in FIG. 1A as needed. Although described below in detail, the apparatus has, as basic hardware components, a light source 1, a light transmitter 3, a light irradiator 4, a holder 5, probes (transducers) 9, a probe supporter 10, an acoustic matching member 11, a scan stage 12, and a stage controller 13. The light source 1 emits light 2. A measurement target is an object 6, and a light absorber 7 generates and propagates a photoacoustic wave 8. An optical controller is denoted by reference numeral 14.

Light 2 emitted from the light source 1 is transmitted to the light irradiator 4 by the light transmitter 3. The light 2 from the light irradiator 4 is radiated through the holder 5 to the object 6 held by the holder 5. The radiated light 2 propagates through the object 6 in a diffusing manner. When a portion of the energy of the propagated light 2 is absorbed by the light absorber 7 such as the blood (which eventually serves as a sound source), the light absorber 7 thermally expands to generate the photoacoustic wave 8. The photoacoustic wave 8 generated inside the object 6 is received by the probes 9 through the holder 5 and the acoustic matching member 11. The light transmitter 3 and the probes 9 move on the scan stage along with the holder 5. The moving coordinate of the light transmitter 3 and the probes 9 are controlled by the stage controller 13.

### (Light Source)

When the object is an organism, the light source 1 radiates pulsed light with a wavelength at which the light is absorbed by a particular one of the components forming the organism. The wavelength of the light used in the present invention preferably allows the light to propagate to the inside of the object. Specifically, when the object is an organism, the wavelength is 600 nm or more and 1,100 nm or less. To efficiently generate the photoacoustic wave, a pulse width is suitably set to 10 to 100 nanoseconds. The light source is preferably a laser that provides a high output. However, a light emitting diode, a flash lamp, or the like may be used instead of the laser. As the laser, various lasers such as a solid laser, a gas laser, a pigment laser, and a semiconductor laser may be used. An irradiation timing, a waveform, an intensity, and the like are controlled by a light source controller. The light source controller may be integrated with the light source. The light source may be provided separately from the photoacoustic apparatus in the present invention.

### (Light Transmitter)

The light transmitter may implement transmission through optical fibers, transmission through a by arm using a plurality of mirrors or prisms, spatial transmission using lenses, mirrors, or diffusers, or a combination thereof. The light from the light source may be input directly to the light transmitter 3 or modified so as to have an appropriate density or shape using lenses or diffusers before being input to the light transmitter.

### (Light Flux Controller)

A light flux controller is not an essential component of the photoacoustic apparatus in the present invention, but will be described below. The light flux controller controls the orientation, spread, shape, and the like of a light flux radiated from the light irradiator 4. Specifically, the light flux controller may include an optical element such as a diffuser, a lens, or a mirror. The light flux controller may be provided between the light source and the light transmitter 3 or between the light transmitter and the light irradiator.

### (Light Irradiator)

The light irradiator is provided in the probe supporter to guide light from the light transmitter to the object through the probe supporter. A material for the light irradiator is suitably glass, resin, or the like. However, any material may be used so long as the material allows light to pass through. Furthermore, an anti-reflection film may be provided on a surface of the light irradiator.

When the object is an organism, specifications for the light source and the light irradiator and a method for controlling the optical controller need to be determined such that the irradiation light satisfies a safety standard. As an example of the safety standard for the human body, a maximum permissible exposure (MPE) is specified (JIS standard C6802 and IEC 60825-1). The MPE defines an upper limit on the quantity of light energy permitted to be radiated for each segment such as the skin or the eye. Therefore, also in the present invention, it is necessary to appropriately control the pulse width of light, an optical-amount distribution and an irradiation density distribution in irradiation light, an irradiation area, the frequency of the pulse, a wavelength of light, a measurement time, and the like. Specifically, the optical controller controls the optical amount such that the maximum optical amount is prevented from exceeding the MPE.

The position of the light irradiator relative to the probe supporter may be changed by the probe supporter, scan stage, and the stage controller in a direction different from an irradiation direction (Z direction in FIG. 1A) of light from the light irradiator. The "different direction" as used herein refers to an X direction or a Y direction in FIG. 1A and is typically within an XY plane. However, a variation in a Z direction is not completely excluded.

### (Optical Controller)

The optical controller controls the optical amount of light radiated from the light irradiator. Specifically, the optical controller controls the optical amount of light radiated from the light irradiator according to the position of the light irradiator relative to the holder in the Z direction in FIG. 1A. This configuration enables a reduction in a variation in the optical amount of light radiated to the object. The optical controller includes a CPU.

### (Object and Light Absorber)

The object and the light absorber form no part of the photoacoustic apparatus in the present invention, but will be described below. The photoacoustic apparatus in the present invention using a photoacoustic effect is mainly intended to take images of blood vessels, diagnose malignant tumors and vascular diseases in human beings and animals, and monitor chemical treatment over time. The light absorber in the object has a relatively high absorption coefficient in the object though the absorption coefficient depends on the wavelength of light used. Specific examples of the light absorber include water, fat, protein, oxygenated hemoglobin, and reduced hemoglobin.

### (Holder)

The holder holds the object. This makes the surface profile of the object stable, facilitating estimation of the optical amount in the object and calculation of a delay experienced by the photoacoustic wave during image reconstruction. A member with a high light transmittance is used as the holder in order to allow the light from the light irradiator to reach the object. Moreover, to allow the photoacoustic wave from the object to pass through, the material has an acoustic impedance close to the acoustic impedance of the object. An example of such a holder may be polymethyl pentene or a rubber sheet. Furthermore, to allow the probes to efficiently receive the photoacoustic wave from the object, the holder and the object are preferably brought into contact with each other via a liquid such as water or a gel.

Preferably, a plurality of holders with different sizes is prepared so as to be changed to one another according to the size of the breast. In this case, the distance from the light irradiator to the holder varies with the type of the holder, and thus, a gain by which the optical amount is multiplied needs to be changed in spite of the same coordinate of the light irradiator. Therefore, it is preferable to provide a mechanism that acquires the type of the holder or an interface via which the user inputs the type and to allow the optical controller to use a different gain table depending on the type of the holder.

### (Probe)

The probes (transducers) receive and transform the photoacoustic wave generated on the surface of the organism and inside the organism into an electric signal. Any probes may be used so long as the probes can receive the photoacoustic wave, for example, probes using a piezoelectric phenomenon, probes using light resonance, probes using a change in electrostatic capacitance, or the like. To shorten the measurement time and improve image quality, desirably a plurality of probes is two- or three-dimensionally arranged or measurement is performed while the probes are being moved by a scan mechanism. To allow the light reflected by the surface of the object or the holder or the light coming out of the object after having scattered through the object to return to the object, a reflection film such as a gold film may be provided on the surface of the probe.

### (Probe Supporter)

The probe supporter maintains the relative positional relations among a plurality of probes. The probe supporter is desirably rigid and a possible material for the probe supporter is, for example, metal. To allow the light reflected by the surface of the object or the holder or the light coming out of the object after having scattered through the object to return to the object, a reflection film such as a gold film may be provided on a surface of the probe supporter that is closer to the object. To allow a photoacoustic signal generated in the object to be received at different angles, a plurality of probes is preferably arranged at different angles. Thus, in the embodiments below, a bowl-shaped probe supporter is used. However, the probe supporter may be shaped like a flat plate. Besides the bowl shape, various shapes such as a semispherical shape, a spherical crown shape, an arc, a dish shape, a part of an ellipsoid, and a shape resulting from a combination of a plurality of planes or curved surfaces may be used so long as the shape allows a plurality of probes to be supported such that directional axes (directions in which the probes exhibit high sensitivity) cross one another to form a high-sensitivity area.

### (Acoustic Matching Member)

The acoustic matching member is arranged to fill the inside of the bowl-shaped probe supporter to connect the holder and the probes together. The acoustic matching member desirably allows the light from the light irradiator to pass through and makes the acoustic impedances of the holder and the probes close to each other. A possible material for the acoustic matching member may be water, a gel, or oil. The object and the holder are arranged on a side of the probe supporter that is closer to the object so as to acoustically connect to the filled acoustic matching member. For example, in an apparatus in which the subject lying on a bed on the subject's stomach with the subject's breast hanging downward and held by the holder, the holder and the object are located above the probe supporter. Therefore, the light irradiator irradiates the object and the holder with light from below the object. The probes receive the photoacoustic wave generated by and propagating from the object.

### (Acquirer)

An acquirer not depicted in the drawings executes predetermined signal processing on electric signals output by the probes to acquire characteristic information. The acquirer includes a signal processor and an information processor. The signal processor digitalizes and amplifies an analog electric signal output from the probe. The signal processor may also execute various correction processes as needed. The information processor performs image reconstruction for the inside of the object using a digital electric signal derived from the photoacoustic wave. At this time, known various techniques such as a phasing addition method and a Fourier domain method may be utilized. The acquirer is implemented using an electric circuit, an information processing apparatus having a processor and a storage member and operating in accordance with a program, or the like.

### (Storage Member)

Any storage member may be used so long as the storage member can store information used for the information processing apparatus. For example, a semiconductor memory or a hard disk is preferable. As described below in detail, the storage member preferably stores a table that holds a gain amount corresponding to the positions of the object (or the holder) and the light irradiator (or the probe supporter) relative to each other. Optical amount control information held in the storage member is not limited to the gain value.

### (Display Member)

A display member not depicted in the drawings displays characteristic information generated by the acquirer as images. Any display member may be used such as a liquid crystal display or an organic EL display. The display member may be configured as a part of the object information acquiring apparatus in the present invention or provided separately from the apparatus.

### (Scan Stage and Stage Controller)

The scan stage scans the probe supporter along with the light transmitter with respect to the holder in a direction different from the direction in which the light is radiated from the light irradiator. The scan stage is controlled by the stage controller. The stage controller one-, two-, or three-dimensionally moves the light transmitter and the probe supporter in order to perform photoacoustic measurement at any positional coordinate in accordance with a predetermined program or the user's specification. Even a scan mechanism that moves only the light irradiator without moving the probe supporter falls within the scope of the present invention because the thickness of the acoustic matching member, through which light passes, varies with the scan position. The scan stage and the stage controller correspond to a scan controller in the present invention.

### [Embodiment 1]

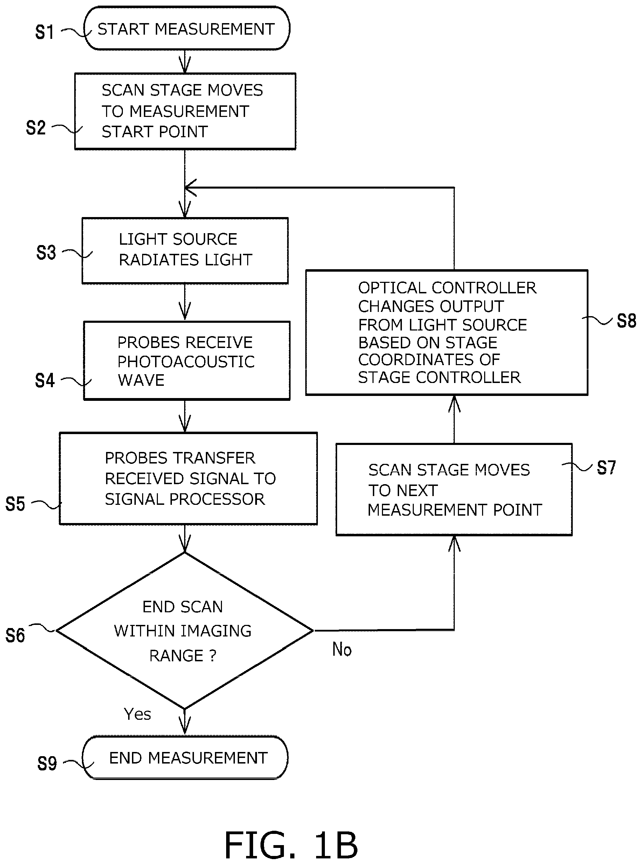

An apparatus in the present embodiment has a configuration in FIG. 1A. In a <state 1> in FIG. 1A, the light irradiator 4 and the probe supporter 10 perform a scan so as to irradiate a central portion of the holder 5 with light. In a <state 2> in FIG. 1A, the light irradiator 4 and the probe supporter 10 perform a scan so as to irradiate a peripheral portion of the holder 5 with light. FIG. 1 is a diagram illustrating a measurement flow of the present embodiment.

The light source 1 is a titanium sapphire laser that outputs pulsed light with a wavelength of 797 nm, an output of 120 mJ, a frequency of 20 Hz and a pulse width of 10 nanoseconds. The light 2 from the light source 1 is transmitted by the light transmitter 3, which is a bundle fiber in which a plurality of optical fibers is bundled. The light 2 from the light transmitter 3 passes through the light irradiator 4 and is radiated to the object 6 through the holder 5. The light irradiator 4 is a polycarbonate plate.

In the <state 1> in FIG. 1A, the central portion of the holder 5 is at a distance of 100 mm from the light irradiator 4 in the Z direction. A Z coordinate of the central portion of the holder surface (that is, a bottom of the bowl) is at a distance of 50 mm from a Z coordinate of an end of the holder surface (that is, an edge of the bowl) . Therefore, in the <state 2> in FIG. 1A, the light irradiator 4 and the holder 5 are at a distance of 150 mm from each other in the Z direction. The <state 1> and the <state 2> in FIG. 1A are schematic block diagram depicting positional relations and are not to the scale of the actual apparatus. When the cup-shaped holder is used as in the present embodiment, the calculation of the optical amount can be simplified by using the center of a circle as a reference. However, even when the holder is not shaped point-symmetrically, the optical amount can be acquired by calculating the optical amount as needed or approximating the shape.

The light having diffused through the object 6 is absorbed by the light absorber 7. Then, the photoacoustic wave 8 is generated by the light absorber 7, propagates through the object 6, the holder 5, and the acoustic matching member 11, and is received by the probes 9. The acoustic matching member 11 is water. The probes 9 are capacitive micro-machined ultrasonic transducers (CMUT). At least some of the probes 9 are arranged on the bowl-shaped probe supporter 10 such that directions in which the probes exhibit the highest sensitivity for reception directivity cross one another. The light transmitter 3 and the probe supporter 10 move in accordance with a scan pattern controlled by the stage controller 13. The scan pattern in the present embodiment allows the light transmitter 3 and the probe supporter 10 to radiate light and receive the photoacoustic wave while spirally moving in an XY plane.

The optical controller 14 controls the output from the light source 1 based on the stage coordinates of the stage controller 13. Specifically, the optical controller 14 makes the output from the light source 1 higher when the light irradiator 4 scans the peripheral portion of the holder as in the <state 2> in FIG. 1A than when the light irradiator 4 scans the central portion of the holder as in the <state 1>. In other words, the optical controller multiplies the optical amount by a larger gain for a longer distance between the light irradiator to the holder surface (that is, the distance that the light passes through the acoustic matching member 11).

Now, output control of the light source by the optical controller 14 will be described. In the present embodiment, light with a wavelength of 797 nm is radiated using the light source 1. Table 1 is a table based on the total optical amount of light having reached the holder after irradiation. Reference character A denotes a distance between the stage coordinate at which the light irradiator moving in the XY plane is located and a foot of a perpendicular from the central portion of the holder surface to the XY plane in which the light irradiator moves.

**[Table 1]**

| A | B | C | D | E |
|---|---|---|---|---|
| STAGE COORDINATE (mm) | TOTAL OPTICAL AMOUNT (mJ) | (*)1 | (*)2 | CORRECTED OPTICAL AMOUNT RATIO |
| | 797nm | 797nm | 797nm | 797nm |
| 0 | 233.91 | 1 | 1 | 1 |
| 25 | 233.64 | 0.99885 | 1.00116 | 1 |
| 50 | 230.66 | 0.98611 | 1.01409 | 1 |
| 75 | 225.29 | 0.96315 | 1.03826 | 1 |
| 100 | 202.98 | 0.86777 | 1.15238 | 1 |

| | | | | |
|---|---|---|---|---|
| (*)1 : OPTICAL AMOUNT RATIO OF EACH STAGE COORDINATE TO STAGE COORDINATE OF 0 (*)2 : RECIPROCAL OF OPTICAL AMOUNT RATIO OF EACH STAGE COORDINATE TO STAGE COORDINATE OF 0 | | | | |

As described above, when the light irradiator is located immediately below the center of the holder (the lowest part of the bowl), in other words, when the value for reference character A is 0 mm, the total optical amount of light reaching the holder becomes a maximum amount. An increase in the value for reference character A increases the distance between the positions of the light irradiator and the holder relative to each other, reducing the total optical amount. Therefore, the value indicated by the reference character A is an indicator reflecting how far the light irradiator and the holder are located away from each other and is also an indicator reflecting the thickness of the acoustic matching member through which light passes before reaching the holder. The attenuation amount of light resulting from absorption and scattering increases in accordance with an increase in this indicator value.

When the probe supporter is located substantially immediately below the holder, the reference is a positional relation in which the light irradiator lies immediately below the center of the holder. However, the present invention is not limited to this. In the present invention, the optical controller performs control so as to make the optical amount smaller when the light irradiator is positioned to correspond to the central portion of the holder than when the light irradiator is positioned to correspond to the peripheral portion of the holder. In other words, the optical controller performs control so as to make the optical amount larger when the light irradiator is positioned to correspond to the peripheral portion of the holder than when the light irradiator is positioned to correspond to the central portion of the holder.

Reference character B denotes the total optical amount (mJ) of light reaching the holder surface when the light irradiator is located at each coordinate. This indicates that the total optical amount of light reaching the holder decreases gradually as the light irradiator is further distanced from the position immediately below the center of the holder. The optical amount ratio of each stage coordinate to a stage coordinate of 0 is denoted by reference character C. For example, for light with a wavelength of 756 nm, the optical amount decays down to approximately 85% when the light irradiator is displaced 100 mm from the center of the holder. Reference character D is the reciprocal of reference character C and denotes a gain corresponding to the stage coordinate. Reference character E denotes the optical amount ratio of each stage coordinate to a stage coordinate of 0 when the light irradiator irradiates the object with light after the optical controller applies the gain for the stage coordinate. The table indicates that the total optical amount is the same in any positional relations between the light irradiator and the holder, reflecting the effect of the present embodiment.

Now, a flow of measurement in the present embodiment will be described using FIG. 1B. First, a manipulator starts measurement (S1). Then, the scan stage 12 moves to a measurement start point (S2). The light source 1 radiates the light 2 (S3). The probes 9 receive the photoacoustic wave 8 from the object 6 (S4). The signal received by the probes 9 is transferred to the signal processor not depicted in the drawings (S5). The system then determines whether or not imaging within a pre-specified range has ended (S6).

When the system determines that the imaging within the pre-specified range has not ended, the scan stage moves to the next measurement point (S7). Then, in the present invention, the optical controller 14 changes the output from the light source 1 based on the stage coordinates of the stage controller 13 (S8) and returns to S3. When the system determines at S6 that the imaging within the pre-specified range has ended, the measurement ends (S9).

As described above, in the present embodiment, the optical controller changes the output from the light source based on the stage coordinates of the stage controller. This enables a reduction in a variation in the optical amount of light radiated to the object in spite of a possible difference in the attenuation amount of light decayed by the acoustic matching member between the light irradiator and the object. As a result, the measurement accuracies of the initial sound pressure distribution and the absorption coefficient distribution are improved.

### [Variation]

In the above-described process flow, the optical-amount controller increases the gain in accordance with an increase in the distance from the stage coordinate of the light irradiator to the position immediately below the center of the holder. This is because the stage coordinate reflects the positions of the holder and the light irradiator relative to each other and can thus be utilized as an indicator for the attenuation amount of light. However, an indicator for the attenuation amount of light other than the stage coordinate may be used. For example, the distance from the light irradiator to the holder may be measured and used as an indicator. In this case, the optical controller increases the gain in accordance with an increase in the distance. The distance can be measured by any technique such as ultrasonic echo measurement, optical imaging means, or a mechanical detecting method.

The optical-amount controller in the present invention is not limited to adjustment using the gain value. For example, the optical amount may be directly specified according to the positions of the holder and the light irradiator relative to each other. Alternatively, the optical amount may be specified using the value of control data inherent in the light source apparatus. The object of the present invention can be achieved using any optical-amount control information that allows the optical amount to be increased in accordance with an increase in the distance between the relative positions and thus with the attenuation amount of light, while allowing optical amount to decrease in accordance with a decrease in the relative value and thus an decrease in the attenuation amount of light.

The technique in the present invention can be utilized even if the light irradiator moves three-dimensionally. In this case, a table may be utilized in which the gain is recorded for each of the X, Y, and Z coordinates. Furthermore, the gain value may be specified for each possible coordinate of the stage or may be the same within a given coordinate range. In the latter case, such a table is preferably used as allows a variation in the total optical amount of light radiated to the object to fall within a predetermined range. The predetermined range is such that, regardless of the position of the irradiator, the total optical amount is 0.9 or more and 1.1 or less when the optical amount obtained when the irradiator is located immediately below the center of the holder is set to 1. Alternatively, a table may be used in which the value of the optical-amount control information (for example, the gain value) changes in steps or curvedly according to the distance between the holder and the irradiator (or the passage distance in the acoustic matching member or the attenuation amount of light) .

### [Embodiment 2]

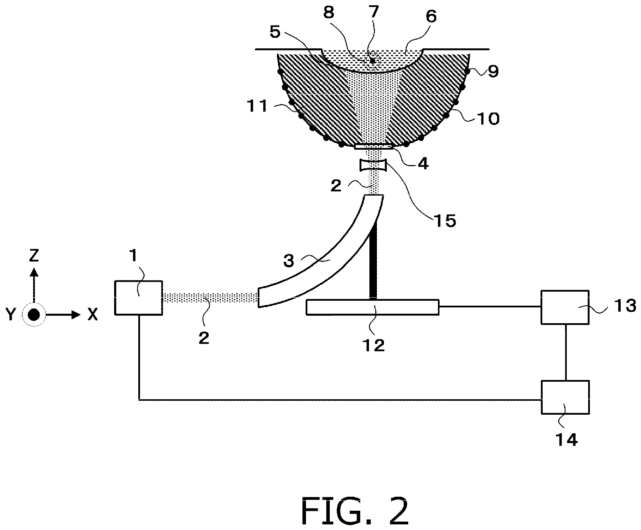

A configuration of the present embodiment will be described using FIG. 2. In FIG. 2, components 1 to 14 are the same as the corresponding components in FIG. 1A, and the light flux controller is denoted by reference numeral 15.

The light flux controller 15 is a recess lens. The light 2 from the light transmitter 3 has the flux thereof spread by the light flux controller 15, passes through the light irradiator 4, and is radiated to the object 6 through the holder 5. When the light is spread by the light flux controller 15, optical paths, in the acoustic matching member 11, of light rays forming the light flux change, and thus, the attenuation amount of light resulting from light absorption also changes. The spread light also changes light density at each position on the object 6 to which the light is radiated. Thus, with the light absorption and the light spread in view, the optical controller 14 in the present embodiment controls the output from the light source 1 so as to decrease the attenuation amount of light among the stage coordinates. Therefore, a flow of measurement in the present embodiment is the same as the flow of measurement in FIG. 1B except for a control method in S8.

As described above, even when the light radiated to the object is spread, the optical controller can control the optical amount by changing the output from the light source. Thus, a variation in the attenuation amount of light in the acoustic matching member can be absorbed to reduce a possible variation in optical amount of light radiated to the object. As a result, the measurement accuracies of the initial sound pressure distribution and the absorption coefficient distribution can be improved. The light radiated to the object need not necessarily be spread but may be narrowed down.

### [Embodiment 3]

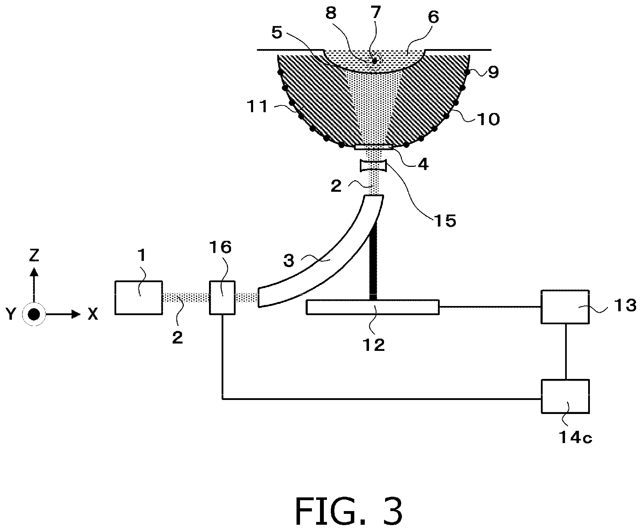

A configuration of the present embodiment will be described using FIG. 3. In FIG. 3, components 1 to 13 and 15 are the same as the corresponding components in FIG. 2. The optical controller is denoted by reference numeral 14c, and a transmittance changer is denoted by reference numeral 16.

The optical controller 14c controls the transmittance of the transmittance changer 16 based on the stage coordinates of the stage controller 13. The transmittance changer 16 is a variable ND filter. However, any mechanism such as a combination of a waveplate and a polarization beam splitter may be used so long as the mechanism allows the transmittance of light to be controlled. The optical controller 14c makes the transmittance of the transmittance changer 16 higher when the light irradiator 4 scans the peripheral portion of the holder 5 than when the light irradiator 4 scans the central portion of the holder 5. This reduces a variation in the optical amount of light radiated to the object 6 among the stage coordinates. A flow of measurement in the present embodiment is basically the same as the flow of measurement in FIG. 1B except for the control in S8. That is, in the present embodiment, the total optical amount is controlled by controlling the transmittance instead of changing the output from the light source.

As described above, in the present embodiment, the optical controller changes the transmittance of the transmittance changer based on the stage coordinates of the stage controller. This allows absorption of a variation in the attenuation amount of light resulting from a variation in the passage distance in the acoustic matching member, enabling a reduction in variation in the optical amount of light radiated to the object. As a result, the measurement accuracies of the initial sound pressure distribution and the absorption coefficient distribution can be improved.

### [Embodiment 4]

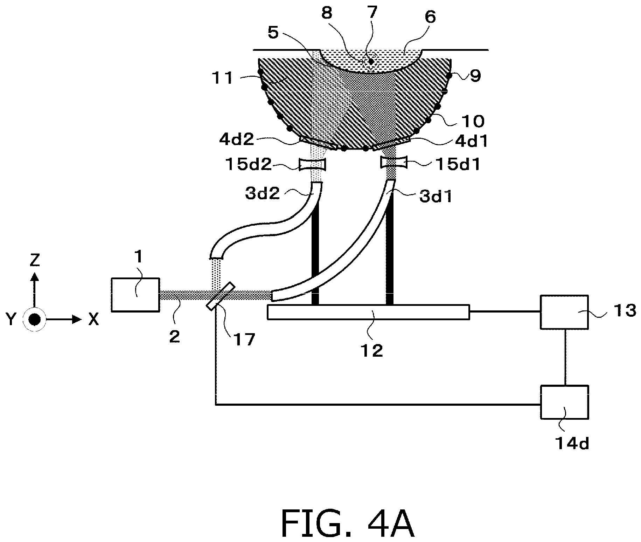

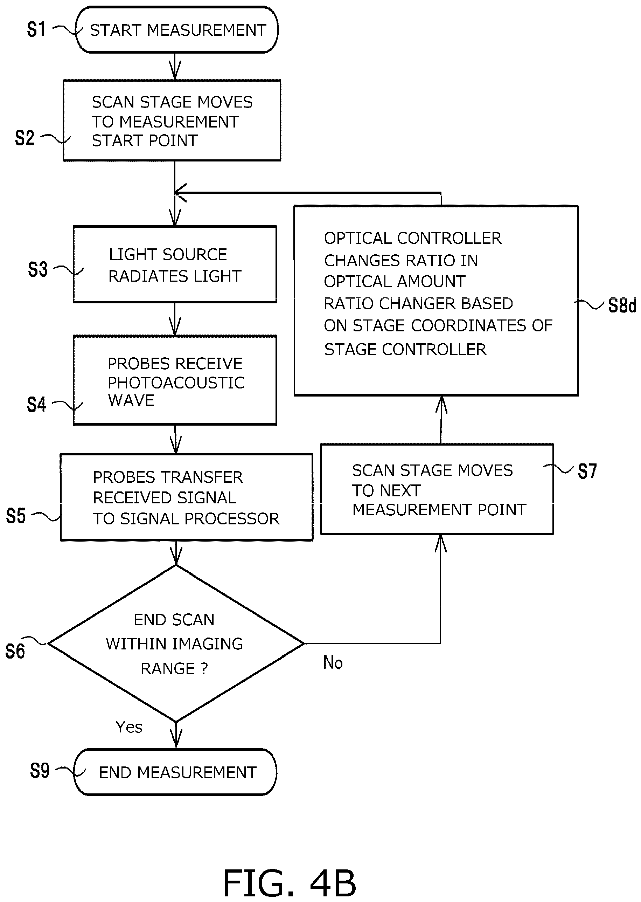

A configuration of the present embodiment will be described. FIG. 4A is a diagram of the configuration of the present embodiment, and FIG. 4B is a diagram illustrating a flow of measurement in the present embodiment. In FIG. 4A, components 1, 2, and 6 to 14 are the same as the corresponding components in FIG. 2 and will thus not be described. The light irradiators are denoted by reference numerals 3d1 and 3d2. The light irradiators are denoted by reference numerals 4d1 and 42. The optical controller is denoted by reference numeral 14d. The light flux controllers are denoted by reference numerals 15d1 ad 15d2. An optical-amount-ratio changer is denoted by reference numeral 17.

The light 2 emitted from the light source 1 is branched into two light rays by the optical-amount-ratio changer 17. The resultant light rays enter light transmitters 3d1 and 3d2, respectively. The optical-amount-ratio changer in the present embodiment is a transmittance varying filter. However, any mechanism such as a combination of a waveplate and a polarization beam splitter may be utilized so long as the mechanism allows a branching ratio of light to be controlled. The light rays emitted from the light transmitters 3d1 and 3d2 pass through light flux controllers 15d1 and 15d2 and light irradiators 4d1 and 4d2, respectively, and are then radiated to the object 6 through the acoustic matching member 11 and the holder 5. However, the light flux controller need not be provided.

The optical controller 14d controls the branching ratio of the optical-amount-ratio changer 17 based on the stage coordinates of the stage controller 13. The optical controller 14d controls the branching ratio of the optical-amount-ratio changer 17 so as to make the optical amount of light radiated to the object 6 larger when the light irradiators 4d1 and 4d2 scan the peripheral portion of the holder 5 than when the light irradiators 4d1 and 4d2 scan the central portion of the holder 5.

FIG. 4B illustrating a flow of measurement in the present embodiment is different from FIG. 1B in S8d. In S8d, the optical controller 14d changes the branching ratio of the optical-amount-ratio changer 17 based on the stage coordinates of the stage controller 13.

As described above, in the present embodiment, the optical controller changes the branching ratio of the optical-amount-ratio changer based on the stage coordinates of the stage controller. Thus, even with a variation in the attenuation amount of light in the acoustic matching member between the light irradiator and the object, a possible variation in the optical amount of light radiated to the object can be reduced. As a result, the measurement accuracies of the initial sound pressure distribution and the absorption coefficient distribution can be improved. In the present embodiment, the example has been described where the light is divided into two light rays. However, the light may be divided into three or more light rays.

### [Embodiment 5]

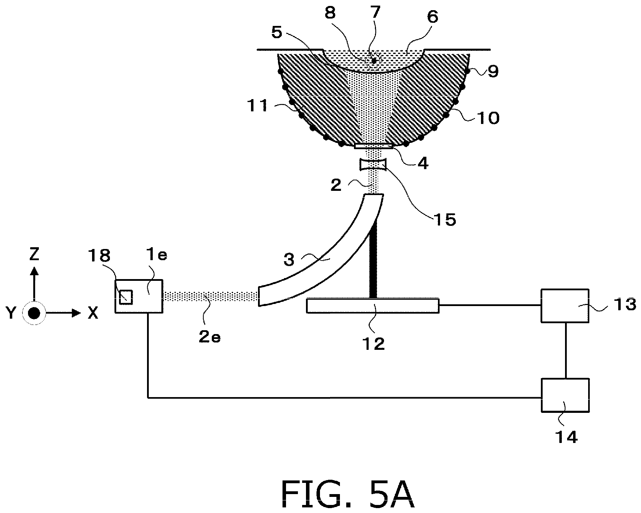

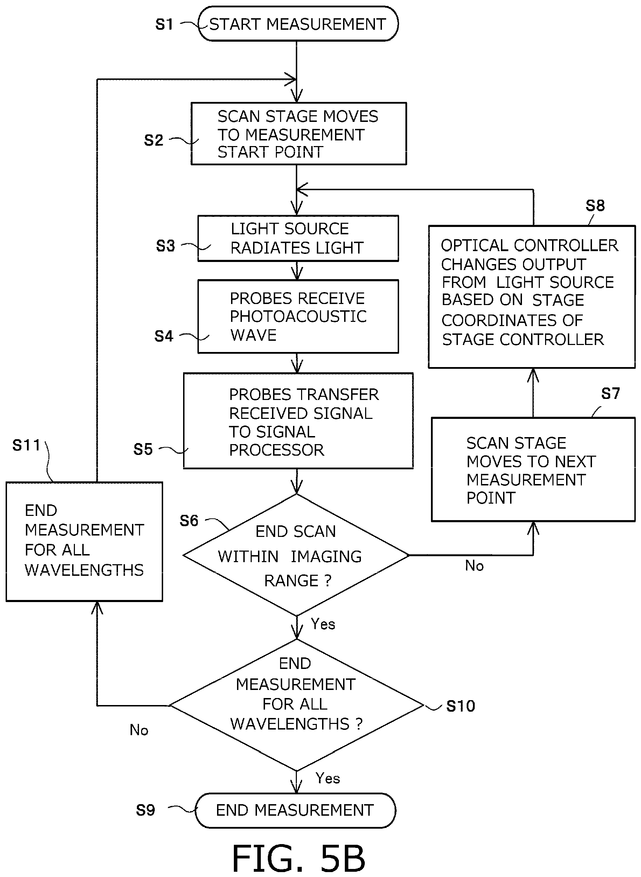

A configuration of the present embodiment will be described. FIG. 5A is a diagram of the configuration of the present embodiment, and FIG. 5B is a diagram illustrating a flow of measurement in the present embodiment. In FIG. 5A, components 3 to 15 are the same as the corresponding components in FIG. 2 and will thus not be described. The light source is denoted by reference numeral 1e. The light is denoted by reference numeral 2e. A wavelength switcher is denoted by reference numeral 18.

The light source 1e is a titanium sapphire laser. The wavelength switcher 18 functions to switch the wavelength of irradiation light. The wavelength switcher 18 is a prism and changes the wavelength of the irradiation light according to the angle of the wavelength switcher 18. In the present embodiment, light 2e emitted by the light source 1e has wavelengths of 797 nm and 756 nm.

Now, an output from the light source 1e controlled by the optical controller 14 will be described with reference to Table 2. As described above, reference character A denotes the distance between the stage coordinate at which the light irradiator moving in the XY plane is located and the foot of the perpendicular from the central portion of the holder surface to the XY plane in which the light irradiator is located. The value for reference character A is an indicator reflecting the distance that light passes through the acoustic matching member before reaching the holder. Reference character B denotes the total optical amount of light reaching the holder surface in conjunction with a scan. Reference character C denotes the ratio of each optical amount to the optical amount obtained when the light irradiator is located immediately below the center of the holder. Reference character D is the reciprocal of reference character C and serves as a basis for calculation of the gain by which the optical amount is multiplied. The effect of optical amount correction in the present embodiment is denoted by reference character E.

**[Table 2]**

| A | B | | C | | D | | E | |
|---|---|---|---|---|---|---|---|---|
| STAGE COORDINATE (mm) | TOTAL OPTICAL AMOUNT (mJ) | | (*)1 | | (*)2 | | CORRECTED OPTICAL AMOUNT RATIO | |
| | 756nm | 797nm | 756nm | 797nm | 756nm | 797nm | 756nm | 797nm |
| 0 | 222.61 | 233.91 | 1 | 1 | 1 | 1 | 1 | 1 |
| 25 | 221.67 | 233.64 | 0.99578 | 0.99885 | 1.00424 | 1.00116 | 1 | 1 |
| 50 | 218.3 | 230.66 | 0.98064 | 0.98611 | 1.01974 | 1.01409 | 1 | 1 |
| 75 | 212.2 | 225.29 | 0.95324 | 0.96315 | 1.04906 | 1.03826 | 1 | 1 |
| 100 | 190.3 | 202.98 | 0.85486 | 0.86777 | 1.16978 | 1.15238 | 1 | 1 |

| | | | | | | | | |
|---|---|---|---|---|---|---|---|---|
| (*)1 : OPTICAL AMOUNT RATIO OF EACH STAGE COORDINATE TO STAGE COORDINATE OF 0 (*)2 : RECIPROCAL OF OPTICAL AMOUNT RATIO OF EACH STAGE COORDINATE TO STAGE COORDINATE OF 0 | | | | | | | | |

When water is used as the acoustic matching member 11, typically a light ray with a wavelength of 756 nm decays at a rate of approximately 2.5%/cm, and a light ray with a wavelength of 797 nm decays at a rate of approximately 2.0%/cm. As a result, even at the same stage coordinate, the degree of decay varies according to the wavelength as represented in Table 2. Thus, when the light source enabling selection from a plurality of wavelengths is used as in the present embodiment, different gain values are used for the respective wavelengths as represented in a section for reference character D. Specifically, the gain value is increased in accordance with an increase in the distance from the stage coordinate to the position immediately below the center of the holder.

An implementation method for controlling the optical amount as described above may be a method in which gain tables for the respective wavelengths and for the respective indicator values are stored in the storage member or mathematical expressions for the respective wavelengths are stored in the storage member. Besides the stage coordinate, the indicator value may be the measured distance between the light irradiator and the holder, the measured distance in the acoustic matching member, control information in a scan program for the probe supporter, or the like.

FIG. 5B illustrating the flow of measurement in the present embodiment is different from FIG. 1B in S10 and S11. Upon determining in S6 that the imaging within the pre-specified range has ended, the system determines whether the measurement has ended for all the wavelengths (S10). When the system determines that the measurement has not ended for all the wavelengths, the wavelength switcher switches to the next wavelength (S11). The system then returns to S2. When the system determines that the measurement has ended for all the wavelengths, the measurement is completed (S9). In this flow, S6 and S10 may be interchanged in order with each other. In that case, when measurement for all the wavelengths is finished at any measurement position (or within a measurement range), the stage controller moves the scan stage to the next measurement position.

In the present embodiment, in S10 and S11, the optical controller changes the output from the light source based on the stage coordinates of the stage controller and the wavelength of light as described above. Then, even with a variation in the attenuation amount of light in the acoustic matching member between the light irradiator and the object, a possible variation in the optical amount of light radiated to the object can be reduced. As a result, the measurement accuracies of the initial sound pressure distribution and the absorption coefficient distribution can be improved. In the present embodiment, the example has been described where the optical controller controls the output from the light source. However, the optical controller may change the transmittance of the transmittance changer or the branching ratio of the optical-amount-ratio changer, similarly to Embodiments 3 and 4.

### [Embodiment 6]

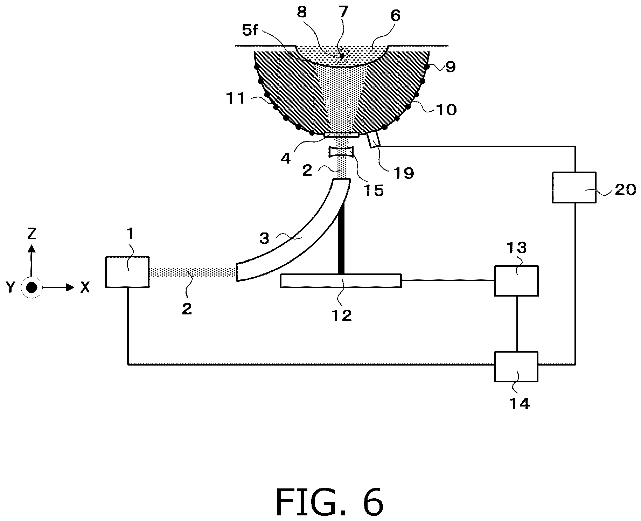

A configuration of the present embodiment will be described using FIG. 6. In FIG. 6, components 1 to 4 and 6 to 15 are the same as the corresponding components in FIG. 2 and will thus not be described. The holder is denoted by reference numeral 5f. An imager is denoted by reference numeral 19. A calculator is denoted by reference numeral 20. The holder 5f is a sheet formed using synthetic rubber as a material. Unlike a cup holding member, the rubber sheet easily extends and contracts.

The imager 19 images the object 6 through the probe supporter 10, the acoustic matching member 11, and the holder 5f. The calculator 20 calculates the distance between the light irradiator 4 and the object 6 based on an output from the imager 19. The calculator 20 calculates the attenuation amount of light in the acoustic matching member 11 for each stage coordinate based on the calculated distance, the light absorption coefficient of the acoustic matching member 11, and the spread of light radiated to the object 6. The optical controller 14 controls, on the basis of the attenuation amount calculated by the calculator 20, the output from the light source 1 so as to reduce a variation in the attenuation amount of light in the acoustic matching member 11 for each stage coordinate. The calculator may exclusively have a function to calculate the distance between the light irradiator and the object. In this case, a table in which the gain value corresponding to the passage distance in the acoustic matching member is recorded may be saved in the storage member, so as to be utilized for subsequent processes.

In a flow of measurement in the present embodiment, the imager and the calculator measure the distance in conjunction with the movement of the probe supporter and the light irradiator for scanning. The optical controller radiates light with the optical amount multiplied by the gain according to the measured distance. Thus, in the present embodiment, the distance measured by the imager and the calculator is used as an indicator reflecting the degree of decay of light.

In the present embodiment, the optical controller changes the output from the light source based on the result of imaging by the imager as described above. Thus, even if the holder is an easily deformable member such as a rubber sheet, a variation in the optical amount of light radiated to the object can be reduced. As a result, the measurement accuracies of the initial sound pressure distribution and the absorption coefficient distribution can be improved. In the present embodiment, the example has been described where the optical controller controls the output from the light source. However, the optical controller may change the transmittance of the transmittance changer or the branching ratio of the optical-amount-ratio changer. Alternatively, measurement may be performed at a plurality of wavelengths as in the case of Embodiment 5.

As described in the embodiments, the present invention enables a reduction in a variation in the amount of light radiated to the object in photoacoustic measurement in which the light irradiator moves.

Furthermore, there may be a computer of a system or apparatus that reads out and executes computer executable instructions recorded on a storage medium (e.g., non-transitory computer-readable storage medium) to perform the functions of the present invention, and a method performed by the computer of the system or apparatus by, for example, reading out and executing the computer executable instructions from the storage medium. The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

## Claims

1. An object information acquiring apparatus comprising:
a light source;
an irradiator configured to irradiate an object with light from the light source;
a holder configured to hold the object;
a probe configured to receive an acoustic wave generated by the object irradiated with the light and output an electric signal;
a scan controller configured to change a relative position of the irradiator to the holder in a direction different from an irradiation direction of the light from the irradiator; and
an acquirer configured to acquire information on the interior of the object based on the electric signal,
wherein the scan controller changes the relative position of the irradiator to the holder such that a thickness of acoustic matching member that acoustically connects the holder and the probe changes according to the relative position in the direction different from the irradiation direction of the light from the irradiator, **characterized by**
an optical controller configured to control an optical amount of light radiated from the irradiator according to the relative position of the irradiator to the holder in the direction different from the irradiation direction of the light from the irradiator.

2. The object information acquiring apparatus according to claim 1, wherein the optical controller changes the optical amount of light radiated from the irradiator so as to increase the optical amount of light radiated from the irradiator in accordance with an increase in a distance between the irradiator and the holder.

3. The object information acquiring apparatus according to claims 1 or 2, wherein the optical controller changes the optical amount of light radiated from the irradiator so as to decrease the optical amount of light radiated from the irradiator in accordance with a decrease in a distance between the irradiator and the holder.

4. The object information acquiring apparatus according to any one of claims 1 to 3, wherein the optical controller changes the optical amount of light radiated from the irradiator so as to increase the optical amount of light radiated from the irradiator in accordance with an increase in an attenuation amount of light in the acoustic matching member through which the light radiated from the irradiator passes.

5. The object information acquiring apparatus according to any one of claims 1 to 3, wherein the optical controller changes the optical amount of light radiated from the irradiator so as to decrease the optical amount of light radiated from the irradiator in accordance with a decrease in an attenuation amount of light in the acoustic matching member through which the light radiated from the irradiator passes.

6. The object information acquiring apparatus according to any one of claims 1 to 5, further comprising a storage that holds information indicative of a relation between optical-amount control information used by the optical controller and the relative position.

7. The object information acquiring apparatus according to claim 6, wherein the storage holds a table that stores the optical-amount control information in association with a position that the irradiator is enabled to take under control of the scan controller.

8. The object information acquiring apparatus according to claims 6 or 7, further comprising a plurality of the holders replaceable with one another according to a size of the object, wherein
the storage holds the optical-amount control information for each of the holders of different sizes.

9. The object information acquiring apparatus according to any one of claims 1 to 8, wherein the optical controller controls an output from the light source.

10. The object information acquiring apparatus according to any one of claims 1 to 8, further comprising a transmittance changer arranged between the light source and the irradiator to change a transmittance of the light, wherein
the optical controller changes the transmittance of the light according to the relative position by using the transmittance changer.

11. The object information acquiring apparatus according to any one of claims 1 to 8, further comprising:
a plurality of the irradiators; and
optical-amount-ratio changers each arranged between the light source and one of the irradiators to change an optical amount ratio of the optical amounts of light transmitted to the respective irradiators, wherein
the optical controller changes the optical amount ratio according to the relative position.

12. The object information acquiring apparatus according to any one of claims 1 to 11, further comprising:
an imager that images the object; and
a calculator that calculates the relative position based on a position of the imaged object.

13. The object information acquiring apparatus according to any one of claims 1 to 12, wherein
the light source radiates light with a plurality of wavelengths, and
the optical controller controls the optical amount for each of the wavelengths.

14. A control method for an object information acquiring apparatus having a light source, an irradiator, an optical controller configured to control an optical amount of light radiated by the irradiator; a holder configured to hold an object, a probe, a scan controller, and an acquirer, the control method comprising:
a step in which the irradiator irradiates the object with light from the light source;
a step in which the probe receives an acoustic wave generated by the object irradiated with the light and outputs an electric signal;
a step in which the scan controller changes a relative position of the irradiator to the holder in a direction different from an irradiation direction of the light from the irradiator; and
a step in which the acquirer acquires information on the interior of the object based on the electric signal,
wherein, in the step of changing the relative position, the scan controller changes the relative position of the irradiator to the holder such that a thickness of acoustic matching member that acoustically connects the holder and the probe changes according to the relative position in the direction different from the irradiation direction of the light from the irradiator, **characterized in that**
in the step of irradiating, the optical controller controls the optical amount of light radiated from the irradiator according to the relative position of the irradiator to the holder in the direction different from the irradiation direction of the light from the irradiator.

## Patentansprüche

1. Objektinformationenbezugsvorrichtung, mit:
einer Lichtquelle;
einer Bestrahlungseinrichtung, die konfiguriert ist, um ein Objekt mit Licht von der Lichtquelle zu bestrahlen;
einer Halterung, die konfiguriert ist, um das Objekt zu halten;
einer Sonde, die konfiguriert ist, um eine akustische Welle zu empfangen, die von dem mit dem Licht bestrahlten Objekt erzeugt wird, und um ein elektrisches Signal auszugeben;
einer Abtaststeuerung, die konfiguriert ist, um eine relative Position der Bestrahlungseinrichtung zur Halterung in eine Richtung zu ändern, die sich von einer Bestrahlungsrichtung des Lichts von der Bestrahlungseinrichtung unterscheidet; und
einer Bezugseinrichtung, die konfiguriert ist, um Informationen über das Innere des Objekts basierend auf dem elektrischen Signal zu erfassen,
wobei die Abtaststeuerung die relative Position der Bestrahlungseinrichtung zur Halterung so ändert, dass sich eine Dicke eines akustischen Anpassungselements, das die Halterung und die Sonde akustisch verbindet, entsprechend der relativen Position in der Richtung, die sich von der Strahlungsrichtung des Lichts von der Bestrahlungseinrichtung unterscheidet, ändert,
**gekennzeichnet durch**
eine Optiksteuerung, die konfiguriert ist, um eine optische Menge von Licht, das von der Bestrahlungseinrichtung abgestrahlt wird, entsprechend der relativen Position der Bestrahlungseinrichtung zur Halterung in der Richtung, die sich von der Bestrahlungsrichtung des Lichts von der Bestrahlungseinrichtung unterscheidet, zu steuern.

2. Objektinformationenbezugsvorrichtung gemäß Anspruch 1, wobei die Optiksteuerung die optische Menge von Licht, das von der Bestrahlungseinrichtung abgestrahlt wird, so ändert, dass die optische Menge von Licht, das von der Bestrahlungseinrichtung abgestrahlt wird, gemäß einer Vergrößerung des Abstands zwischen der Bestrahlungseinrichtung und der Halterung erhöht wird.

3. Objektinformationenbezugsvorrichtung gemäß Anspruch 1 oder 2, wobei die Optiksteuerung die optische Menge von Licht, das von der Bestrahlungseinrichtung abgestrahlt wird, so ändert, dass die optische Menge von Licht, das von der Bestrahlungseinrichtung abgestrahlt wird, gemäß einer Verringerung des Abstands zwischen der Bestrahlungseinrichtung und der Halterung verringert wird.

4. Objektinformationenbezugsvorrichtung gemäß einem der Ansprüche 1 bis 3, wobei die Optiksteuerung die optische Menge von Licht, das von der Bestrahlungseinrichtung abgestrahlt wird, so ändert, dass die optische Menge von Licht, das von der Bestrahlungseinrichtung abgestrahlt wird, gemäß einer Erhöhung eines Dämpfungsausmaßes von Licht in dem akustischen Anpassungselement, das das von der Bestrahlungseinrichtung abgestrahlte Licht durchläuft, erhöht wird.

5. Objektinformationenbezugsvorrichtung gemäß einem der Ansprüche 1 bis 3, wobei die Optiksteuerung die optische Menge von Licht, das von der Bestrahlungseinrichtung abgestrahlt wird, so ändert, dass die optische Menge von Licht, das von der Bestrahlungseinrichtung abgestrahlt wird, gemäß einer Abnahme eines Dämpfungsausmaßes von Licht in dem akustischen Anpassungselement, das das von der Bestrahlungseinrichtung abgestrahlte Licht durchläuft, verringert wird.

6. Objektinformationenbezugsvorrichtung gemäß einem der Ansprüche 1 bis 5, weiterhin mit einem Speicher, der Informationen hält, die eine Beziehung zwischen den von der Optiksteuerung verwendeten Steuerinformationen der optischen Menge und der relativen Position angeben.

7. Objektinformationenbezugsvorrichtung gemäß Anspruch 6, wobei der Speicher eine Tabelle hält, die die Steuerinformationen der optischen Menge in Verbindung mit einer Position speichert, die die Bestrahlungseinrichtung unter der Kontrolle der Abtaststeuerung übernehmen kann.

8. Objektinformationenbezugsvorrichtung gemäß Anspruch 6 oder 7, weiterhin mit einer Vielzahl der Halterungen, die entsprechend einer Größe des Objekts gegeneinander austauschbar sind, wobei
der Speicher die Steuerinformationen der optischen Menge für jede der Halterungen unterschiedlicher Größe hält.

9. Objektinformationenbezugsvorrichtung gemäß einem der Ansprüche 1 bis 8, wobei die Optiksteuerung eine Ausgabe von der Lichtquelle steuert.

10. Objektinformationenbezugsvorrichtung gemäß einem der Ansprüche 1 bis 8, weiterhin mit einer Transmissionsgradänderungseinrichtung, die zwischen der Lichtquelle und der Bestrahlungseinrichtung angeordnet ist, um einen Transmissionsgrad des Lichts zu ändern, wobei
die Optiksteuerung die Transmission des Lichts entsprechend der relativen Position unter Verwendung der Transmissionsgradänderungseinrichtung ändert.

11. Objektinformationenbezugsvorrichtung gemäß einem der Ansprüche 1 bis 8, weiterhin mit:
einer Vielzahl der Bestrahlungseinrichtungen; und
Änderungseinrichtungen der optischen Mengenverhältnisse, die jeweils zwischen der Lichtquelle und einer der Bestrahlungseinrichtungen angeordnet sind, um ein optisches Mengenverhältnis der zu den jeweiligen Bestrahlungseinrichtungen übertragenen optischen Lichtmengen zu ändern, wobei
die Optiksteuerung das optische Mengenverhältnis entsprechend der relativen Position ändert.

12. Objektinformationenbezugsvorrichtung gemäß einem der Ansprüche 1 bis 11, weiterhin mit:
einer Bildgebungseinrichtung, die das Objekt abbildet; und
einer Berechnungseinrichtung, die die relative Position basierend auf einer Position des abgebildeten Objekts berechnet.

13. Objektinformationenbezugsvorrichtung gemäß einem der Ansprüche 1 bis 12, wobei
die Lichtquelle Licht mit einer Vielzahl von Wellenlängen abstrahlt, und
die Optiksteuerung die optische Menge für jede der Wellenlängen steuert.

14. Steuerungsverfahren für eine Objektinformationenbezugsvorrichtung mit einer Lichtquelle, einer Bestrahlungseinrichtung, einer Optiksteuerung, die konfiguriert ist, um eine optische Menge von Licht zu steuern, die von der Bestrahlungseinrichtung abgestrahlt wird; einer Halterung, die konfiguriert ist, um ein Objekt zu halten, einer Sonde, einer Abtaststeuerung, und einer Bezugseinrichtung, wobei das Steuerungsverfahren umfasst:
einen Schritt, in dem die Bestrahlungseinrichtung das Objekt mit Licht von der Lichtquelle bestrahlt;
einen Schritt, in dem die Sonde eine akustische Welle empfängt, die von dem mit dem Licht bestrahlten Objekt erzeugt wird, und ein elektrisches Signal ausgibt;
einen Schritt, in dem die Abtaststeuerung eine relative Position der Bestrahlungseinrichtung zur Halterung in einer Richtung ändert, die sich von einer Bestrahlungsrichtung des Lichts von der Bestrahlungseinrichtung unterscheidet; und
einen Schritt, in dem die Bezugseinrichtung basierend auf dem elektrischen Signal Informationen über das Innere des Objekts bezieht,
wobei die Abtaststeuerung in dem Schritt des Änderns der relativen Position die relative Position der Bestrahlungseinrichtung zur Halterung so ändert, dass sich eine Dicke des akustischen Anpassungselements, das die Halterung und die Sonde akustisch verbindet, entsprechend der relativen Position in der Richtung ändert, die sich von der Strahlungsrichtung des Lichts vom Bestrahlungseinrichtung unterscheidet,
**dadurch gekennzeichnet, dass**
in dem Schritt des Bestrahlens die Optiksteuerung die optische Menge von Licht, das von der Bestrahlungseinrichtung abgestrahlt wird, gemäß der relativen Position der Bestrahlungseinrichtung zur Halterung in einer Richtung, die sich von der Bestrahlungsrichtung des Lichts von der Bestrahlungseinrichtung unterscheidet, steuert.

## Revendications

1. Appareil d'acquisition d'informations d'objet, comprenant :
une source de lumière ;
un dispositif d'exposition à un rayonnement configuré pour exposer un objet à un rayonnement de lumière provenant de la source de lumière ;
un dispositif de maintien configuré pour maintenir l'objet ;
une sonde configurée pour recevoir une onde acoustique générée par l'objet exposé au rayonnement de la lumière et pour délivrer un signal électrique ;
un dispositif de commande de balayage configuré pour modifier une position relative du dispositif d'exposition à un rayonnement et du dispositif de maintien dans une direction différente d'une direction d'exposition au rayonnement de la lumière provenant du dispositif d'exposition à un rayonnement ; et
un dispositif d'acquisition configuré pour acquérir des informations concernant l'intérieur de l'objet sur la base du signal électrique,
dans lequel le dispositif de commande de balayage modifie la position relative du dispositif d'exposition à un rayonnement et du dispositif de maintien de façon à modifier une épaisseur d'un élément d'adaptation acoustique qui assure la liaison acoustique entre le dispositif de maintien et la sonde conformément à la position relative dans la direction différente de la direction d'exposition au rayonnement de la lumière provenant du dispositif d'exposition à un rayonnement, **caractérisé par** un dispositif de commande optique configuré pour commander une quantité optique de lumière de rayonnement provenant du dispositif d'exposition à un rayonnement conformément à la position relative du dispositif d'exposition à un rayonnement et du dispositif de maintien dans la direction différente de la direction d'exposition au rayonnement de la lumière provenant du dispositif d'exposition à un rayonnement.

2. Appareil d'acquisition d'informations d'objet selon la revendication 1, dans lequel le dispositif de commande optique modifie la quantité optique de lumière de rayonnement provenant du dispositif d'exposition à un rayonnement de façon à augmenter la quantité optique de lumière de rayonnement provenant du dispositif d'exposition à un rayonnement conformément à une augmentation d'une distance entre le dispositif d'exposition à un rayonnement et le dispositif de maintien.

3. Appareil d'acquisition d'informations d'objet selon les revendications 1 ou 2, dans lequel le dispositif de commande optique modifie la quantité optique de lumière d'exposition provenant du dispositif d'exposition à un rayonnement de façon à diminuer la quantité optique de lumière de rayonnement provenant du dispositif d'exposition à un rayonnement conformément à une diminution d'une distance entre le dispositif d'exposition à un rayonnement et le dispositif de maintien.

4. Appareil d'acquisition d'informations d'objet selon l'une quelconque des revendications 1 à 3, dans lequel le dispositif de commande optique modifie la quantité optique de lumière de rayonnement provenant du dispositif d'exposition à un rayonnement de façon à augmenter la quantité optique de lumière de rayonnement provenant du dispositif d'exposition à un rayonnement conformément à une augmentation d'une quantité d'atténuation de lumière dans l'élément d'adaptation acoustique à travers lequel passe la lumière de rayonnement provenant du dispositif d'exposition à un rayonnement.

5. Appareil d'acquisition d'informations d'objet selon l'une quelconque des revendications 1 à 3, dans lequel le dispositif de commande optique modifie la quantité optique de lumière de rayonnement provenant du dispositif d'exposition à un rayonnement de façon à diminuer la quantité optique de lumière de rayonnement provenant du dispositif d'exposition à un rayonnement conformément à une diminution d'une quantité d'atténuation de lumière dans l'élément d'adaptation acoustique à travers lequel passe la lumière de rayonnement provenant du dispositif d'exposition à un rayonnement.

6. Appareil d'acquisition d'informations d'objet selon l'une quelconque des revendications 1 à 5, comprenant en outre un élément de mémorisation qui maintient des informations représentant une relation entre des informations de commande de quantité optique utilisées par le dispositif de commande optique et la position relative.

7. Appareil d'acquisition d'informations d'objet selon la revendication 6, dans lequel l'élément de mémorisation maintient une table qui mémorise les informations de commande de quantité optique en association avec une position que le dispositif d'exposition à un rayonnement peut prendre sous la commande du dispositif de commande de balayage.

8. Appareil d'acquisition d'informations d'objet selon les revendications 6 ou 7, comprenant en outre une pluralité des dispositifs de maintien pouvant être remplacés les uns les autres conformément à une taille de l'objet, dans lequel :
l'élément de mémorisation mémorise les informations de commande de quantité optique pour tous les dispositifs de maintien de tailles différentes.

9. Appareil d'acquisition d'informations d'objet selon l'une quelconque des revendications 1 à 8, dans lequel le dispositif de commande optique commande une sortie de la source de lumière.

10. Appareil d'acquisition d'informations d'objet selon l'une quelconque des revendications 1 à 8, comprenant en outre un organe de modification de transmittance disposé entre la source de lumière et le dispositif d'exposition à un rayonnement pour modifier une transmittance de la lumière, dans lequel :
le dispositif de commande optique modifie la transmittance de la lumière conformément à la position relative au moyen de l'organe de modification de transmittance.

11. Appareil d'acquisition d'informations d'objet selon l'une quelconque des revendications 1 à 8, comprenant en outre :
une pluralité des dispositifs d'exposition à un rayonnement ; et
des dispositifs de modification de rapport de quantité optique disposés individuellement entre la source de lumière et l'un des dispositifs d'exposition à un rayonnement pour modifier un rapport de quantité optique des quantités optiques de lumière émises vers les dispositifs d'exposition à un rayonnement respectifs, où
le dispositif de commande optique modifie le rapport de quantité optique conformément à la position relative.

12. Appareil d'acquisition d'informations d'objet selon l'une quelconque des revendications 1 à 11, comprenant en outre :
un dispositif de formation d'image qui forme une image de l'objet ; et
un calculateur qui calcule la position relative sur la base d'une position de l'objet mis en image.

13. Appareil d'acquisition d'informations d'objet selon l'une quelconque des revendications 1 à 12, dans lequel :
la source de lumière émet une lumière ayant une pluralité de longueurs d'onde, et
le dispositif de commande optique commande la quantité optique pour chacune des longueurs d'onde.

14. Procédé de commande d'un appareil d'acquisition d'informations d'objet comportant une source de lumière, un dispositif d'exposition à un rayonnement, un dispositif de commande optique configuré pour commander une quantité optique de lumière émise par le dispositif d'exposition à un rayonnement ; un dispositif de maintien configuré pour maintenir un objet, une sonde, un dispositif de commande de balayage et un dispositif d'acquisition, le procédé comprenant :
une étape durant laquelle le dispositif d'exposition à un rayonnement expose l'objet à un rayonnement de lumière provenant de la source de lumière ;
une étape durant laquelle la sonde reçoit une onde acoustique générée par l'objet soumis à une exposition au rayonnement de la lumière et délivre un signal électrique ;
une étape durant laquelle le dispositif de commande de balayage modifie une position relative du dispositif d'exposition à un rayonnement et du dispositif de maintien dans une direction différente d'une direction d'exposition au rayonnement de la lumière provenant du dispositif d'exposition à un rayonnement ; et
une étape durant laquelle le dispositif d'acquisition acquiert des informations concernant l'intérieur de l'objet sur la base du signal électrique,
dans lequel, à l'étape de modification de la position relative, le dispositif de commande de balayage modifie la position relative du dispositif d'exposition à un rayonnement et du dispositif de maintien de façon à modifier une épaisseur d'élément d'adaptation acoustique qui assure une liaison acoustique entre le dispositif de maintien et la sonde conformément à la position relative dans la direction différente de la direction d'exposition au rayonnement de la lumière provenant du dispositif d'exposition à un rayonnement,
**caractérisé en ce que**
à l'étape d'exposition à un rayonnement, le dispositif de commande optique commande la quantité optique de lumière de rayonnement provenant du dispositif d'exposition à un rayonnement conformément à la position relative du dispositif d'exposition à un rayonnement et du dispositif de maintien dans la direction différente de la direction d'exposition au rayonnement de la lumière provenant du dispositif d'exposition à un rayonnement.
